(19) Europäisches Patentamt
European Patent Office
Office européen des brevets

(11) EP 0 792 647 A1

(12) **EUROPEAN PATENT APPLICATION**

(43) Date of publication:
**03.09.1997 Bulletin 1997/36**

(51) Int. Cl.$^6$: **A61K 35/80**, A61K 31/40

(21) Application number: **96102885.9**

(22) Date of filing: **27.02.1996**

(84) Designated Contracting States:
**DE**

(71) Applicant: **BOEHRINGER MANNHEIM GMBH
68298 Mannheim (DE)**

(72) Inventors:
- **König, Bernhard, Dr., Dipl.-Biologe
82335 Berg (DE)**
- **Weidle, Ulrich, Dr., Dipl.-Biochemiker
80336 München (DE)**
- **Krell, Hans-Willi, Dr., Dipl.Biologe
82377 Penzberg (DE)**
- **Kilpert, Claus, Dr., Dipl.-Chemiker
68305 Mannheim (DE)**
- **Knipp, Bernhard, Dr., Chemiker
51515 Kürten (DE)**

# (54) Porphyrins as urokinase-type plasminogen activator receptor antagonists

(57) The present invention relates to the use of porphyrins as drugs, especially as drugs for urokinase and its receptor (uPAR) mediated diseases. The porphyrines show uPA-uPAR antagonist activity.

EP 0 792 647 A1

## Description

The present invention relates to the use of porphyrins as drugs, especially as drugs for urokinase and its receptor (uPAR) mediated diseases. The porphyrines show uPA-uPAR antagonist activity.

Plasminogen activation catalyzed by the serine protease uPA (urokinase-type plasminogen activator) is involved in a variety of physiological and pathological processes such as the proteolytic degradation of extracellular matrix necessary for cellular invasion, migration and tissue remodelling. A well characterized uPA cell surface receptor (uPAR) showing high-affinity binding for uPA ($K_D$ = $10^{-10}$ - $10^{-9}$ M) plays a key role in these processes by potentiating plasmin generation in the pericellular environment [Nielsen, L.S., Kellerman, G.M., Behrendt, N., Picone, R., Danø, K. and Blasi, F.: A 55,000-60,000 Mr receptor protein for urokinase-type plasminogen activator. Identification in human tumor cell lines and partial purification, J. Biol. Chem., 263, 2358, 1988] [Ellis, V., Behrendt, N. and Danø, K.: Plasminogen activation by receptor-bound urolcinsase: A kinetic study with both cell-associated and isolated receptor. J. Biol. Chem. 266, 12752, 1991]. Although matrix metalloproteases (e.g. collagenases) and other proteolytic cascades might cooperate to achieve extracellular matrix degradation the uPA /uPAR system is probably central to the overall process.

The uPA/uPAR system has been shown to be implicated in a variety of invasive biological processes such as tumor metastasis, trophoblast implantation, inflammation and angiogenesis. Therefore, uPA receptor antagonists are able to block tumor invasiveness, metastasis and angiogenesis. Formulations containing uPAR antagonists represent novel therapeutic treatments for a number of highly invasive and metastasizing cancers where uPA and uPAR have been found to be consistently present at the invasive foci of tumors:[ Danø K., Grøndahl-Hansen, J., Eriksen, J., Nielsen, B.S., Rømer, J. and Pyke, C.: The receptor for urokinase plasminogen activator: Stromal cell involvement in extracellular proteolysis during cancer invasion, in *Proteolysis and Protein Turnover*, Barrett, A.J. and Bond, J., Eds., Portland Press, London, 1994,239]; (e.g. breast, lung, colon, ovarian cancer). In addition to cancer, other diseases mediated by cell-surface activity of uPA are addressed by uPAR antagonists. Inhibitors of plasmin generation by receptor bound uPA therefore have mechanism-based tumoristatic, anti-invasive, anti-metastatic, anti-angiogenic, anti-arthritic, anti-inflammatory, anti-osteoporotic, anti-retinopathic and contraceptive activities. These compounds could be applied preferentially via the oral route, but also by i.v. or i.m. injections, nasal spays or any other conventionally used application.

A description of uPAR is disclosed in WO 90/12091, Plough et al., J. Biol. Chem. 268 (1993) 17539, Ronne et al., J. Immunol. Methods 167 (1994) 91. Peptide or Antibody inhibitors of uPAR are disclosed in WO 94/28014 and DK 831/94, respectively. uPA-Inhibitors are disclosed in EP 568 289.

The inhibition of uPA dependent phenomena can principally be approached in two ways: Either by direct inhibition of the proteolytic activity or by inhibition of uPA receptor binding. The latter strategy has the potential of achieving greater specificity since inhibition might be localized to the pericellular environment.

A bacteriophage display technique and protein engineering have recently been used to discover peptidic and site specific uPAR antagonists [Goodson et al., PNAS 91 (1994) 7129 and Stratton Thomas et al., Prot. Eng. 5 (1995) 463, respectively].

The invention concerns the use of porphyrine compounds and their derivates for the preparation of medicaments with potent antagonistic activity.

Porphyrine derivates are partially hydrated porphyrines, e.g. Chlorines, Bacteriochlorines, Corphines, Pyrrocorphines, Phlorines and Porphyrinogens. Other derivates are metallo-porphyrines like Turacin and N-Alkyl porphyrins like N-Methyl-protoporphyrine. Within the meaning of the present invention, the term "porphyrine derivatives" should also enbrace compounds known as phtalocyanines and porphycenes (US 5,409,900).

Porphyrins are as well known substance class. References regarding their structures, synthesises and sources can be obtained e.g. by CD-Römpp Chemie Lexikon, 1st edition, Georg Thieme Verlag, Stuttgart, 1995. Further syntheses are described in R.B. Woodward et al., Tetrahedron 1990, 46, 7599; A.R. Battersby, E. McDonald, Acc. Chem. Res. 1979 12, 14 and B. Franck, Angew. Chemie 1982, 94, 327.

Surprisingly it was found that compounds of general formula I act as antagonists of the uPA/uPAR system.

Formula I:

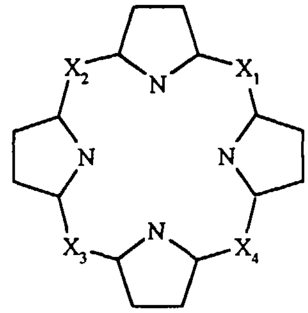

General formula I shows only the necessary atomic backbone structure (pharmacophor). Possible double bonds or aromatic systems are not indicated in this formula but known to a person skilled in the art. $X_1$ to $X_4$ represent valency bonds, N or C atoms with the provisio that the number of backbone atoms is in the range between 3 and 5. All backbone atoms of general formula I may be substituted or part of aromatic systems (e.g. as in phtalocyanines). Substances of general formula I can be obtained by chemical synthesis or isolation of porphyrins (e.g. chlorophyll) and their derivatives and degradation products from natural sources. A more detailed general structure is given in general formula II

Formula II:

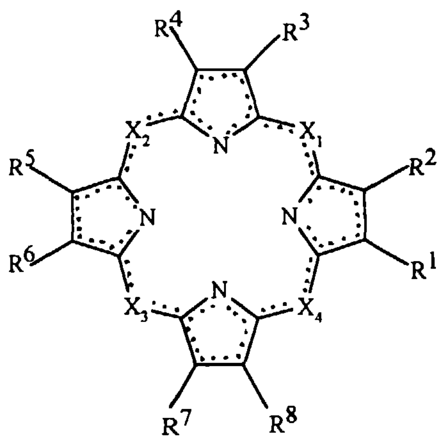

wherein

$R^1$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^2$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^1$ and $R^2$ together can optionally respresent a five or six membered aromatic or saturated carbo- or heterocycle which may optionally bear one or more substituents selected from unbranched or branched alkyl- or alkenyl group, five and six membered aromatic or saturated carbo- and heterocycles all of which can be independently substituted by one or more hydroxygroups, alkylgroups, carboxyl groups and its esters and sulfonic acid groups and their esters,

$R^3$ represents a hydrogen, a formyl group, a carboxyl group and its esters, a unbranched or branched alkyl- or alkenyl group, five and six membered aromatic or saturated carbo- and heterocycles all of which may optionally bear

one or more substituents selected from hydroxy groups, alkyl groups, carboxyl groups and its esters and sulfonic acid groups and their esters,

$R^4$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^3$ and $R^4$ together can optionally respresent a five or six membered aromatic or saturated carbo- or heterocycle whichmay optionally bear one or more substituents selected from unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$R^5$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^6$ represtens a hydrogen, a formyl group, a carboxyl group and its esters, an unbranched or branched alkyl or alkenyl group, five and six membered aromatic or saturated carbo- and heterocycles all of which may optionally bear one or more substituents selected form hydroxy groups, alkyl groups, carboxyl groups and its esters and sulfonic acid groups and their esters, $R^5$ and $R^6$ together can respresent a five or six membered aromatic or saturated carbo- or heterocycle, all of which can be independently substituted by one or more a unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$R^7$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^8$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^7$ and $R^8$ together can optionally respresent a five or six membered aromatic or saturated carbo- or heterocycle whichmay optionally bear one or more substituents selected from unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$X_1$ represents a valence dash, a nitrogen, a one-carbon- or two-carbon-backbone where the carbons are independently substituted by a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$X_2$, $X_3$ and $X_4$ respectively have, independently from each other, the same meaning as $X^1$ with the provisio that the sum of backbone atoms of $X_1$ to $X_4$ is in the range of 3 to 5;

the nitrogen atoms of the porphyrine system may independently bear a hydrogen, a methyl- or ethyl group or may function as ligands for a cation.
the dotted line represents bonds which may have single or double bond character.
Substances of general formula II or a pharmaceutical acceptable salt thereof act as uPAR antagonists.

The terminus 'alkyl group' stands for a branched or unbranched $C_1$-$C_6$ alkyl group, the terminus 'alkenyl group' stands for a branched or unbranched $C_2$-$C_6$ alkenyl group. 'Heterocycle' means cycles where one, two or three carbons are independently from each other substituted by O, S or N respectively.

Compounds (II) or their salts can be solvated, especially hydrated. Hydration may happen during preparation and storage.

Surprisingly, it was found that natural extracts, obtained from lyophilisated crude algae extracts show an uPA/uPAR inhibition effect.
The crude extract can be purified by HPLC, using a acetonitril/water gradient.
The active principle shows an absorption spectra with a maximum in the range of between 400-450 nm. The molecular mass in larger than 500 u, preferably less than 2000 u.

Another method, alternative to chemical synthesis, to obtain porphyrines of general formula I is to isolate them from natural sources. Such sources are for example algae which are obtainable from public microorganism collections like the American Type Culture Collection (ATCC), Deutsche Sammlung für Mikroorganismen (DSM) or Stammsammlung Göttingen, Germany.

Isolation methods for algae are given in Algenreinkulturen, VEB Gustav Fischer, Jena, 1954, Typical growing media are known in the literature (e.g. A. Zehnder, E.O. Hughes (1958) Can. J. Microbiol 4, 309-408). The growing conditions are sterile and usually under continuous illumination, e.g. Phillips "warm white" fluorescent lamp TLE 22W/33 (average illumination ca. 30 $Wm^{-2}$). The growing temperature is usually in the range between 15° and 35°C, preferrably at 20°C. The pH-value of growing cultures is maintained between 7 and 10, preferrably at 8.5. To avoid too alkaline media during growth gaseous $CO_2$ may be used. The algae should be continuously in contact with sufficient nutrients. Such can be obtained by stirring or shaking. The time interval for fermentation is usually in the range of 8 to 30 days.

The processing of the harvested algae is done in a conventional manner. The porphyrines are extracted by organic solvents, e.g. the lyphilisate of the cells is extracted with methanol. A description for further extraction and purification of porphyrines is examplified in the examples.

The porphyrine compounds of the general formula (I) are tested in three different test systems. An ELISA-Test was used to defect the inhibition of the uPA/uPAR binding. An enzyme test was used to detect inhibtion of urokinase activity and a cellular assay using NIH 3T3 (from ATCC) cells transfected with a human urokinase receptor was obtained. The results of test show that the porphyrine of the general formula (I) work well as uPAR antagonists.

The invention concerns pharmaceutical agents comprising new compounds of general formula (I) and (II). In order to produce pharmaceutical agents, the compounds of the general formula (I) or (II) are mixed in a know manner with suitable pharmaceutical carrier substances, aromatics, flavourings and dyes and are formed for example into tablets or coated tablets or they are suspended or dissolved in water or an oil such as e.g. olive oil with addition of appropriate auxiliary substances.

The substance of the general formula (I) or (II) can be administered orally or parenterally in a liquid or solid form. Water is preferably used as the medium which contains the stabilizing agents, solublizers and/or buffers which are usually used for injection solutions. Such additives are for example tartrate or borate buffers, ethanol, dimethylsulfoxide, complexing agents (such as ethylenediaminetetraacetic acid), high molecular polymers (such as liquid polyethylene oxide) for the regulation of the viscosity or polyethylenen derivatives of sorbitol anhydrides.

Solid carrier substances are e.g. starch, lactose, mannitol, methylcellulose, talcum, highly dispersed silicic acid, higher molecular fatty acids (such as stearic acid), gelatin, agar-agar, calcium phosphate, magnesium stearate, animal and vegetable fats or solid high molecular polymers (such as polyethylene glycols). Suitable formulations for the oral application can if desired contain flavourings and sweeteners.

The administered dose depends on the age, the health and the weight of the recipient, the extent of the disease, the type of treatments which are possibly beeing carried out concurrently, the frequency of the treatment and the type of the desired effect. The daily dose of the active compound is usually 0.1 to 50 mg/kg body weight. Normally 0.5 to 40 and preferably 1.0 to 20 mg/kg/day in one or several applications per day are effective in order to obtain the desired results.

Three different assays are used to test different substances regarding their ability to block uPAR. An alternate test which can be used to determine the inhibiting activity of porphyrins in the uPA/uPAR-system is a chemical crosslinking assay (Nielson et., J. Biol. Chem. 263 (1988), 2385) or an 8-anilino-1-naphtalene fluorescence assay (Plough et al., Biochemistry 33 (1994), 8991). To select for such substances which inhibit mostly uPAR and not uPA another assay was done in parallel being basicly the uPAR assay without using the receptor. As a third assay, a cellular test was done where again the activity of uPAR is obtained.

Example 1

This test is described by P. Rettenerger et al. in Biol. Chem. Hoppe-Seyler 376, pp. 587-594 (1995). An alternate ELISA-test is described in Behrendt et al., Meth. Enzymol. 233 (1993), 207-222.

ELISA-Test: Inhibition of human urokinase (uPA) binding to its specific receptor (uPAR). The assays are performed in Microtiterplates (96 wells). The following solutions are used:
washing buffer: PBS-buffer (w/o $Mg^{2+}Ca^{2+}$) + 0,05% Tween 20; uPAR-solution 100 ng/ml (10 ng/well)
blocking solution 2% skimmed milk powder (Merck) in washing buffer
incubation buffer 0.5% skimmed milk powder in PBS uPA-solution 10 ng/well detection solutions (per microfilter

plate):

(1) 6 ml (100 mM Tris-Cl pH 7.5 + 0.15% Tween 80) + 1.5 ml (10 µg) Plasminogen in aqua bidest.

(2) 6 ml (100 mM Tris-Cl pH 7.5 + 0.15% Tween 80) + 1.5 ml (7.5 mg) Chromozyme PL in aqua bidest.

The detection solution must be continously stirred.

Testing substances: the testing substances are solved in DMSO. They are used in the test system with a highest concentration of 100 µg/ml. Dissolutions are prepared using PBS.

Three controlls are performed:

a) positive controll: using 2% DMSO in PBS
b) negative controll: assay w/o receptor
c) inhibition controll: Inhibition ($IC_{95}$ at 0.25 mg/ml) with Dextranesulfate (MW = 500.000)

Incubation is done as follows:

Each well is incubated by 10 ng uPAR for 1 hour at roomtemperature (RT). After washing once each well is incubated for half an hour (37°C) with 200 µl/well blocking solution. After triple washing testing substance solution or controll solutions are added (50 µl/well and incubated for 15 min at RT. An additional 50 µl of uPA solution (10 ng in incubation buffer) are added. After 1 hour at RT a triple washing is performed.

For detection, the following procedure is used:

Incubation 70 µl each of solution (1) and (2) at RT. After 20 min. a yellow color will be visible (the positive control reads an extinction of 1 after 40 min.). The detection is performed at 405 mm (reference is 490 mm) using a Dynatech MR 7000 ELISA reader.

To obtain the percentage of inhibition the following formula is used:

% Inhibtion = 100-100* (Extinction (Test) - Extinction (Neg. contr.)/(Extinction (Pos. contr.) - Extinction (Neg. contr.))

Example 2

ENZ: Urokinase-activity-assay (uPA not bound to uPAR)

This assay is identical to the uPA/uPAR assay of example one except that no uPAR is used, i.e. no incubation of the wells with uPAR and the following washing steps is necessary.

Example 3

Medium:

DMEM (MEM dulbecco/Boehringer) with $NaHCO_3$ and 4.5 g/l Glucose

add:

10% FCS (Seromed)
2 mM L-Glutamine
1 mM Na-Pyruvat
100 U/ml Penicillin
100 µg/ml Streptomycin
1 µg/ml G 418 (Geneticin)
sterilize all supplements by filtration

Coating wells of cellcultureplate with fibronectin:

Take a 1 mg/ml fibronectin solution (in sterile water) and dilute an appropriate aligot with sterile PBS to a final concentration of 50 µg/ml. Coat the 96-well plate with 200 µl//well. Incubate for about 45 min. at room temperature. Remove the solution carefully. The wells should not be allowed to dry, use immediately. Prepare the cells during the 45 min. of incubation with fibronectin.

NIH3T3 CH1 (15.10)-cells are grown to confluency in T 175 cell-culture flasks in the above described medium. Remove the media and rinse the cells with PBS, add 2 ml of Trypsin / EDTA solution for cell detachment. Stop trypsin activity by addition of approx. 6 ml medium . Take an aliquot of the cells to determine the cellnumber and adjust the celldensity to 8 x $10^5$/ml with medium.
Take the fibronectin coated 96 well palate and spread 100 µl cell-suspension/well, let the cells grow to confluency over night.

Discard the medium carefully and wash the cells by addition of 200 µl medium (without supplements) / well and again discarding the medium. Add 200 µl 2% skimmed milk in medium. Incubate for 30 minutes at roomtemperatur. Wash twice with 200 µl medium (w supplements) / well carefully (to remove unbound skimmed milk). Preincubate with 50 µl testcompound diluted in medium, respectively medium only for the controls for 20 minutes; add 50 µl uPA-solution [200 ng/ml]. Incubate for 60 minutes at roomtemperature; wash carefully for 3 times with medium (200 µl/well). Add 100 µl detection solution/well (see Example 1). Measure at a wavelength of 405 nm (reference: 490 nm) with an ELISA-Reader. The yellow colour will appear after about 45 mins.

Example 4

The results of synthetic or commercially available porphyrins and their derivates in the different testing systems are shown in the following table.

| compound | $IC_{50}$ (µg/ml) ELISA | $IC_{50}$ (µg/ml) ENZ | $IC_{50}$ (µg/ml) cells |
|---|---|---|---|
| meso-tetra-(4-Sulfonatophenyl)-Porphine Dihydrochlorid | 0,2 | 0,63 | 19,9 |
| meso-tetra-(4-Carboxyphenyl)-Porphine | 0,21 | 0,68 | 24,2 |
| Mesoporphyrin IX Dihydrochloride | 0,23 | 1,2 | 1,57 |
| Chlorin e6 | 0,31 | 1,4 | 7,7 |
| N-Methyl Protoporphyrin IX | 0,4 | N.D. | N.D. |
| Coproporphyrin III tetraethylester | 0,52 | 73 | 95 |
| Hematoporphyrin D [a)] | 0,52 | 3,1 | 1,4 |
| Deuteroporphyrin IX 2,4 Disulfonic Acid dimethylester | 1,27 | 2,45 | N.D. |
| Al Phtalocyanine Tetrasulfonate | 3,7 | 2,3 | N.D. |
| Deuteroporphyrin IX 2,4-Bis Glycol | 5,3 | 2,7 | N.D. |

[a)] The isolation of Hematoporphyrin D is described for example in R.L. Lipson et al., J. Natl. Cancer Inst. 26 (1961) 1.

Comparative Example A:

No significant uPA/uPAR antagonist behaviour is found, testing Bilirubine which is a open chained porphyrine derivate. Even at 300 µg/ml an inhibition in ELISA of only 11% is observed.

Example 5:

An alternate source of porphyrins and their derivates of general formula (I) are algae.
The following algae are used to examplify their utility as sources of porphyrins and derivates Lagerheimia ciliata, Haemotococcus capensis, Zyguema cylindricum, Cyclotella cryptica, Chlorella fusca.
The following procedure was obtained to obtain algae extracts with uPA/uPAR antagonist activity.
Crude extracts are solved in DMSO. The concentration was adjusted to 100 mg/ml.
Insolvable parts are separated by carbrifugation. The clear solution was used in HPLC.
HPLC experimental parameters:

0.5 mg-10 mg algae extract in DMSO; column: HI-PAK ODS AB, 5µm; length: 250 mm; ∅: 4-8 mm; C18; Fa. Bischoff, 71229 Leonberg, Germany gradient: 70%-100% Acetotutril; time: 30 minutes; eluent: Acetonitril/water 0.1%

TFA; 1-2 ml/min 25°C; detector: PDA 996 (Waters, USA)

The obtained fractions were dried, dissolved in DMSO and tested. UV/Vis-Maxima of fractions which show activity in the uPA/uPAR ELISA test. For comparison the respective abs. maxima of chlorin e6 are given in addition.

| algae | maxima [nm] |
|---|---|
| Lagerheimia ciliata | 419+667 |
| Haematococus capensis | 413+667; 433+652 |
| Zygnema cylindricum | 414+667; 419+667 |
| Cyclotella cryptica | 414+667; 419+564, 428+558, 438+334 |
| Chlorella fusca | 409+652; 414+667 |
| Chlorin e6 | 409+652 |

In addition to the UV/Vis Spectra, the mass spectra obtained from active fractions confirm that the antagonlstic substances are porphyrin or derivates thereof. The following molecular weights were obtained: 653.4; 635.2; 625.3; 607.3; 608.0; 593.1.

$IC_{50}$ values in the ELISA-test are as follows:

| algae | $IC_{50}$ |
|---|---|
| Lagerheimia ciliata | 3 μg/ml |
| Haematococus capensis | 5 μg/ml |
| Zygnema cylindricum | 10 μg/ml |
| Cyclotella cryptica | 10 μg/ml |
| Chlorella fusca | 20 μg/ml |

The algae extracts contain only 1-10% of chlorophyll-degradation products. The IC50 value of the pure porphyrins and derivates obtained by algae extracts is therefore only 1/10 to 1/100 of the above identified $IC_{50}$ values of algae extracts.

## Claims

1. Use of a compound obtained by algae extracts for preparation of a medicament with uPA receptor antagonist activity.

2. Use of a compound according to claim 1 where the compound has a molecular weight higher than 500.

3. Use of a compound according to one of the claims 1 or 2 where the compound has a maximum in the absorption spectrum in the range between 400 and 450 nm.

4. Use of a compound of general formula I for preparation of a medicament with uPA receptor antagonist activity

Formula I:

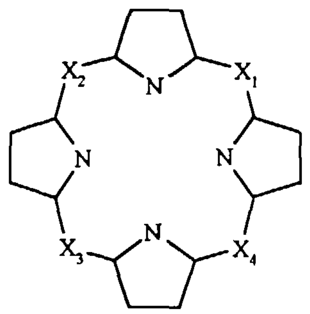

wherein only the necessary atomic backbone structure is shown.

**5.** Use of a compound of general formula II for preparation of a medicament with uPA receptor antagonist activity

Formula II:

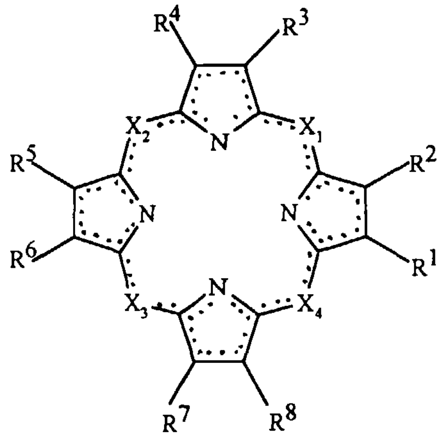

wherein

$R^1$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^2$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^1$ and $R^2$ together can optionally respresent a five or six membered aromatic or saturated carbo- or heterocycle which may optionally bear one or more substituents selected from unbranched or branched alkyl- or alkenyl group, five and six membered aromatic or saturated carbo- and heterocycles all of which can be independently substituted by one or more hydroxygroups, alkylgroups, carboxyl groups and its esters and sulfonic acid groups and their esters,

$R^3$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its

esters and sulfonic acid groups and their esters,

$R^4$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^3$ and $R^4$ together can optionally respresent a five or six membered aromatic or saturated carbo- or heterocycle whichmay optionally bear one or more substituents selected from unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$R^5$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^6$ represtens a hydrogen, a formyl group, a carboxyl group and its esters, an unbranched or branched alkyl- or alkenyl group, five and six membered aromatic or saturated carbo- and heterocycles all of which may optionally bear one or more substituents selected form hydroxy groups, alkyl groups, carboxyl groups and its esters and sulfonic acid groups and their esters, $R^5$ and $R^6$ together can respresent a five or six membered aromatic or saturated carbo- or heterocycle, all of which can be independently substituted by one or more a unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$R^7$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$R^8$ represents a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters, $R^7$ and $R^8$ together can optionally respresent a five or six membered aromatic or saturated carbo- or heterocycle whichmay optionally bear one or more substituents selected from unbranched or branched alkyl- or alkenyl group, five and six membered aromatic or saturated carbo- and heterocycles all of which can be independently substituted by one or more hydroxy groups, alkyl groups, carboxyl groups and its esters and sulfonic acid groups and their esters,

$X_1$ represents a valence dash, a nitrogen, a one-carbon- or two-carbon-backbone where the carbons are independently substituted by a hydrogen, a formyl group, a carboxyl group and its esters, a unbranched or branched alkyl- or alkenyl group, five and six membered aromatic or saturated carbo- and heterocycles all of which may optionally bear one or more substituents selected from hydroxy groups, alkyl groups, carboxyl groups and its esters and sulfonic acid groups and their esters,

$X_2$, $X_3$ and $X_4$ respectively have, independently from each other, the same meaning as $X^1$ with the provisio that the sum of backbone atoms of $X_1$ to $X_4$ is in the range of 3 to 5;

the nitrogen atoms of the porphyrine system may independently bear a hydrogen, a methyl- or ethyl group or may function as ligands for a cation

and their pharmaceutically acceptable salts.

European Patent Office

# EUROPEAN SEARCH REPORT

Application Number
EP 96 10 2885

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | PROC. NATL. ACAD. SCI., vol. 91, no. 15, 19 July 1994, USA, pages 7129-7133, XP000574761 ROBERT J. GOODSON ET AL.: "High-affinity urokinase receptor antagonists..." --- | | A61K35/80 A61K31/40 |
| D,A | BIOCHEMISTRY, vol. 33, no. 30, 2 August 1994, USA, pages 8991-8997, XP000574762 MICHAEL PLOUG ET AL.: "Ligand interaction between urokinase-type plasminogen activator and its receptor ..." --- | | |
| A | COMP. BIOCHEM. PHYSIOL., vol. 102b, no. 1, 1992, SOUTHAMPTON, GB, pages 163-167, XP000196211 HIROYUKI SUMI ET AL.: "Fibrinolysis relating substances in marine creatures" ----- | | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| BERLIN | 17 July 1996 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)